(19) Europäisches Patentamt
European Patent Office
Office européen des brevets

(11) Veröffentlichungsnummer: **0 055 431**
**B1**

(12) **EUROPÄISCHE PATENTSCHRIFT**

(45) Veröffentlichungstag der Patentschrift: **18.04.84**

(51) Int. Cl.³: **C 07 D 211/46** // C07H5/06

(21) Anmeldenummer: **81110570.9**

(22) Anmeldetag: **18.12.81**

(54) **Verfahren zur Herstellung von 1,5-Didesoxy-1,5-imino-D-glucitol und dessen N-Derivaten.**

(30) Priorität: **30.12.80 DE 3049446**

(43) Veröffentlichungstag der Anmeldung: **07.07.82 Patentblatt 82/27**

(45) Bekanntmachung des Hinweises auf die Patenterteilung: **18.04.84 Patentblatt 84/16**

(84) Benannte Vertragsstaaten: **AT BE CH DE FR GB IT LI NL SE**

(56) Entgegenhaltungen:
**DE - A - 2 738 717**
**DE - A - 2 758 025**
**DE - A - 2 830 469**

(73) Patentinhaber: **BAYER AG, Konzernverwaltung RP Patentabteilung, D-5090 Leverkusen 1 Bayerwerk (DE)**

(72) Erfinder: **Köbernick, Wolfgang, Dr., Claudiusweg 3, D-5600 Wuppertal 1 (DE)**

**EP 0 055 431 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 1,5-Didesoxy-1,5-imino-D-glucitol und dessen N-Derivaten

Die Erfindung betrifft ein neues Verfahren zur Herstellung von 1,5-Didesoxy-1,5-imino-D-glucitol (Desoxynojirimycin) und dessen N-Derivaten.

Diese Verbindungen sind potente Inhibitoren für α-Glycosidasen, insbesondere für Disaccharidasen (vgl. DE-OS 2 758 025). Die am Stickstoff unsubstituierte Verbindung ist in der Literatur als Desoxynojirimycin bekannt.

Es ist bekannt, dass man 1,5-Didesoxy-1,5-imino-D-glucitol erhält, wenn man 6-Amino-6-desoxy-L-sorbose in wässriger Lösung mit Platinkatalysator nach Adams hydriert [H. Paulsen, J. Sangster und K. Heyns, Chem. Ber. 100 (1967), 802].

Nachteilig an diesem Verfahren ist die Verwendung der sehr labilen freien Base der 6-Amino-6-desoxy-L-sorbose. Aus der freien 6-Amino-6-desoxy-L-sorbose bildet sich immer durch Eliminierung ein substituiertes Pyridinderivat als Nebenprodukt. Weitere Nachteile dieser Verfahrensweise sind die hohen Herstellungskosten, die bei der Verwendung von Platin anfallen und die Bildung von grösseren Mengen (ca. 30%) des isomeren 1,5-Didesoxy-1,5-imino-L-iditols.

Weiterhin ist aus der DE-A-1 28 30 459 bekannt, 6-Amino-6-desoxy-L-sorbose oder deren N-Derivate mit Borhydriden z.B. Natriumborhydrid zu Desoxynojirimycin oder seinen N-Derivaten zu reduzieren.

Es wurde nun gefunden, dass man eine solche Reduktion ganz besonders vorteilhaft mit Dimethylaminoboran durchführen kann. Dabei wird im Vergleich zu $NaBH_4$ nicht nur eine bessere Ausbeute erhalten, sondern auch die Aufarbeitung des Reaktionsgemisches gestaltet sich durch Wegfall einer Ionenaustauscher-Behandlung wesentlich einfacher.

Die Erfindung betrifft daher ein Verfahren zur Herstellung von 1,5-Didesoxy-1,5-imino-D-glucitol (Desoxynojirimycin) und dessen N-Derivaten der allgemeinen Formel I

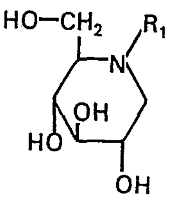

(I)

worin in

$R_1$ Wasserstoff, einen Alkylrest mit 1 bis 30 C-Atomen, der gegebenenfalls substituiert sein kann durch Hydroxy, Alkoxy mit 1 bis 4 C-Atomen, Acyloxy,

wobei der Acylrest von aliphatischen Carbonsäuren mit 1 bis 7 C-Atomen, aromatischen Carbonsäuren, insbesondere Phenylcarbonsäure, die im Phenylrest durch Hydroxy, Halogen, $C_1$- bis $C_4$-Alkyl, $C_1$- bis $C_4$-Alkoxy, Nitro und Amino substituiert sein können, heterocyclische Carbonsäuren, die sich von 5- oder 6-gliedrigen Heterocyclen ableiten, die 1–3 Heteroatome aus der Reihe N, O und S enthalten und im heterocyclischen Ring durch $C_1$- bis $C_4$-Alkyl, Chlor, Brom und Amino substituiert sein können, abgeleitet ist; Amino, Mono- und Dialkylamino mit vorzugsweise 1 bis 4 Kohlenstoffatomen je Alkylrest, Monoacylamino, wobei Acyl die vorstehend genannte Bedeutung hat, Mercapto, $C_1$- bis $C_4$-Alkylthio, Halogen, $C_1$- bis $C_4$-Alkylcarbonyl, Carboxy, Nitro, Cyan, Formyl, Sulfo, Heterocyclyl der vorstehend genannten Definition, $C_3$- bis $C_7$-Cycloalkyl und gegebenenfalls durch Halogen, $C_1$- bis $C_4$-Alkyl, $C_1$- bis $C_4$-Alkoxy, Nitro, Cyano oder Carboxy substituiertes Phenyl, oder einen Alkenyl-, Alkinyl- oder einen Alkadienylrest mit 2 bis 18 C-Atomen darstellt, das dadurch gekennzeichnet ist, dass man Verbindungen der allgemeinen Formel II

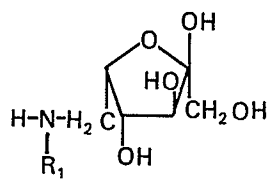

. HX (II)

worin $R_1$ die obengenannte Bedeutung hat und HX eine starke anorganische oder organische Säure ist, mit Dimethylaminoboran in Wasser, Wasser-Lösungsmittelgemischen oder in organischen Lösungsmitteln reduziert.

Erfindungsgemäss bedeutet $R_1$ vorzugsweise einen Alkylrest mit 1 bis 18 C-Atomen, der jeweils wie genannt substituiert sein kann oder einen Alkenyl-, Alkinyl oder Alkadienylrest mit 2 bis 10 C-Atomen.

Halogen ist insbesondere Fluor, Chlor und Brom. Beispiele für Heterocyclen in den vorstehend genannten Definitionen sind Phthalimido, Pyridinyl, Thienyl, Furyl, Isoxazolyl, Thiazolyl, Glucopyranosyl, Ribofuranosyl und Oxiranyl.

Vorzugsweise sind die Alkylreste $R_1$ unsubstituiert oder durch Hydroxy, $C_1$- bis $C_4$-Alkoxy, Mercapto, $C_1$- bis $C_4$-Alkylthio, Halogen, Nitro, Amino, $C_1$- bis $C_4$-Alkylamino oder $C_1$- bis $C_6$-Alkylcarbonylamino substituiert, wobei der Substituent vorzugsweise nicht an dem Ringstickstoff benachbarten Kohlenstoffatom steht.

Die starke Säure HX in der allgemeinen Formel II kann beispielsweise $H_2SO_4$, HCl oder $HClO_4$ sein. Vorzugsweise stellt HX Salzsäure dar. Im Prinzip ist aber jede starke Säure, die das Ausgangsprodukt, das Reaktionsprodukt und die Reduktion mit dem komplexen Borhydrid nicht in unerwünschter Weise beeinflusst, geeignet.

Die erfindungsgemässe Reduktion wird in Wasser oder Wasser-Lösungsmittelgemischen oder in organischen Lösungsmitteln durchgeführt. Als Lösungsmittel kommen hierbei niedere Alkohole, vorzugsweise Methanol, Ethanol und

Isopropanol in Frage. Ebenso können Ether oder Etheralkohole wie Ethylenglykolmono- oder -dimethylether eingesetzt werden. Das zur Reduktion verwendete $HN(CH_3)_2BH_3$ wird vor der eigentlichen Reduktion dem Lösungsmittel(gemisch), in dem die Reduktion erfolgt, zugesetzt. Hierbei entsteht durch Reaktion mit dem Lösungsmittel ein komplexes Gemisch von Borhydriden, wobei einzelne Hydridionen durch die Alkyl-, Alkyloxy- oder Alkoxyetherreste aus dem Lösungsmittel ersetzt sein können.

In das so erhaltene Reagenz wird der zu reduzierende Aminozucker eingetragen.

Danach wird für einige Zeit, zweckmässig für 30 bis 150 Minuten, vorzugsweise ca. 1 Stunde auf etwa 60–100°C, vorzugsweise 60–80°C, erwärmt.

Es hat sich als zweckmässig erwiesen, die Zugabe des Dimethylaminoborans zu dem Lösungsmittel(gemisch) bei Temperaturen von etwa −80 bis 100°C, vorzugsweise jedoch bei 0 bis 25°C zu betreiben. Ausserdem ist es bei der erfindungsgemäss durchzuführenden Reduktion von Vorteil, wenn dem vorgelegten Dimethylaminoboran ein basischer Hilfsstoff zugesetzt wird, der in der Lage ist, die Säure HX zu binden und dadurch die Menge an eingesetztem Dimethylaminoboran zu reduzieren. Solche basischen Hilfsstoffe sind beispielsweise NaOH, KOH, $Na_2CO_3$ oder organische Basen wie Di- oder Triethylamin. Die Menge an solchen basischen Hilfsstoffen liegt zweckmässig bei oder etwas über der stöchiometrischen Menge, die zur Bindung der Säure HX benötigt wird.

Das eingesetzte Molverhältnis zwischen Aminozucker und Dimethylaminoboran sollte mindestens 1:0,25 betragen, um eine quantitative Hydrierung zu gewährleisten.

Die Isolierung des Reaktionsproduktes kann direkt aus der eingedampften Reaktionslösung erfolgen, vorzugsweise wird jedoch die Reaktionslösung durch Behandlung mit basischem und nötigenfalls mit saurem Ionenaustauscher entmineralisiert.

Die entsprechenden Desoxynojirimycine werden in sehr guter Ausbeute und hoher Reinheit erhalten.

Überraschenderweise verläuft die erfindungsgemässe Reduktion so stereospezifisch, dass das unerwünschte Nebenprodukt (1,5-Didesoxy-1,5-imino-L-iditol) nur noch in Spuren im Reaktionsansatz nachgewiesen werden konnte.

Beispiele

Beispiel 1

Desoxynojirimycin (1,5-Didesoxy-1,5-imino-D-glucitol)

50 g (0,23 Mol) 6-Amino-6-desoxy-L-sorbose-Hydrochlorid werden in 500 ml dest. Wasser gelöst und innerhalb von einer Stunde unter Rühren bei einer Temperatur von 5°C zu einer Lösung von 11,2 g Dimethylaminoboran in 500 ml dest. Wasser gegeben. Der Ansatz wird eine Stunde bei Raumtemperatur und eine Stunde bei 50°C gerührt, mit 5 ml Triethylamin versetzt und dann über eine Säule mit 800 ml stark basischem Ionenaustauscher (Lewatit MP 500 $OH^{\ominus}$-Form) gegeben. Der Austauscher wird mit dest. Wasser nachgewaschen und der gesammelte Ablauf wird am Rotationsverdampfer zum Sirup aufkonzentriert. Der aufkonzentrierte Sirup wird bei 50°C unter Zugabe von viel Ethanol kristallisiert. Die Kristallsuspension wird abgekühlt, abgesaugt und das Kristallisat wird im Vakuumtrockenschrank getrocknet.

Ausbeute: 30 g ≙ 80% der Theorie.

Schmelzpunkt: 192–193°C.

Beispiel 2

Desoxynojirimycin (Vergleich) (1,5-Didesoxy-1,5-imino-D-glucitol)

25 g (0,115 Mol) 6-Amino-6-desoxy-L-sorbose-Hydrochlorid werden in 200 ml dest. Wasser gelöst und unter Rühren bei 5°C zu einem Gemisch von 4,8 g $NaBH_4$, 250 ml Ethanol/Wasser 1:1 und 16,2 ml Triethylamin gegeben. Man rührt eine Stunde bei Raumtemperatur und eine Stunde bei 50°C nach und gibt den Reaktionsansatz über eine Säule mit 400 ml stark basischem Ionenaustauscher (Lewatit MP 500 $OH^{\ominus}$-Form). Der Austauscher wird mit dest. Wasser nachgewaschen und das gesammelte Eluat wird am Rotationsverdampfer zum Sirup eingeengt. Der Sirup wird mit 200 ml dest. Wasser aufgenommen und über eine Säule mit 400 ml saurem Ionenaustauscher (Lewatit S 100 $H^{\oplus}$-Form) gegeben. Die Säule wird mit dest. Wasser gespült und das Produkt wird mit 10%igem Ammoniakwasser eluiert. Der ammoniakalische Ablauf wird gesammelt und am Rotationsverdampfer zum Sirup eingeengt. Der Sirup wird in der Wärme mit viel Ethanol kristallisiert, die Kristallsuspension wird abgekühlt, abgesaugt und das Kristallisat wird im Vakuumtrokkenschrank getrocknet.

Ausbeute: 14 g ≙ 74% der Theorie.

Schmelzpunkt: 192–193°C.

Beispiel 3

N-Hydroxyethyl-1-desoxynojirimycin

20 g (0,077 Mol) 6-[(2-Hydroxyethyl)-amino]-6-desoxy-L-sorbose-Hydrochlorid werden in 200 ml dest. Wasser gelöst und unter Rühren innerhalb von 1 Stunde bei 5°C zu einer Lösung von 4,5 g Dimethylaminoboran in 200 ml dest. Wasser getropft. Man lässt eine Stunde bei Raumtemperatur nachrühren, erwärmt eine Stunde auf 50°C, versetzt mit 2 ml Triethylamin und gibt dann den Ansatz über eine Säule mit stark basischem Ionenaustauscher (Lewatit MP 500 $OH^{\ominus}$-Form). Der Austauscher wird mit dest. Wasser nachgewaschen und der gesammelte Ablauf wird am Rotationsverdampfer aufkonzentriert. Der Sirup wird in der Wärme durch Zugabe von viel Ethanol kristallisiert. Das abgesaugte Kristallisat wird im Vakuumtrockenschrank getrocknet.

Ausbeute: 9,7 g ≙ 61% der Theorie.

Schmelzpunkt: 143–145,5°C.

## Patentansprüche

1. Verfahren zur Herstellung von 1,5-Didesoxy-1,5-imino-D-glucitol und dessen N-Derivaten der allgemeinen Formel I

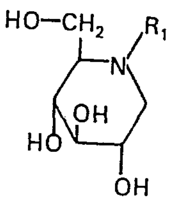

(I)

worin

$R_1$ Wasserstoff, einen Alkylrest mit 1 bis 30 C-Atomen, der gegebenenfalls substituiert sein kann durch Hydroxy, Alkoxy mit 1 bis 4 C-Atomen, Acyloxy, wobei der Acylrest von aliphatischen Carbonsäuren mit 1 bis 7 C-Atomen, aromatischen Carbonsäuren, insbesondere Phenylcarbonsäure, die im Phenylrest durch Hydroxy, Halogen, $C_1$- bis $C_4$-Alkyl, $C_1$- bis $C_4$-Alkoxy, Nitro und Amino substituiert sein können, heterocyclische Carbonsäuren, die sich von 5- oder 6-gliedrigen Heterocyclen ableiten, die 1–3 Heteroatome aus der Reihe N, O und S enthalten und im heterocyclischen Ring durch $C_1$- bis $C_4$-Alkyl, Chlor, Brom und Amino substituiert sein können, abgeleitet ist; Amino, Mono- und Dialkylamino mit vorzugsweise 1 bis 4 Kohlenstoffatomen je Alkylrest, Monoacylamino, wobei Acyl die vorstehend genannte Bedeutung hat, Mercapto, $C_1$- bis $C_4$-Alkylthio, Halogen, $C_1$- bis $C_4$-Alkylcarbonyl, Carboxy, Nitro, Cyan, Formyl, Sulfo, Heterocyclyl der vorstehend genannten Definition, $C_3$- bis $C_7$-Cycloalkyl und gegebenenfalls durch Halogen, $C_1$- bis $C_4$-Alkyl, $C_1$- bis $C_4$-Alkoxy, Nitro, Cyano oder Carboxy substituiertes Phenyl, oder einen Alkenyl-, Alkinyl- oder einen Alkadienylrest mit 2 bis 18 C-Atomen darstellt, dadurch gekennzeichnet, dass man Verbindungen der allgemeinen Formel II

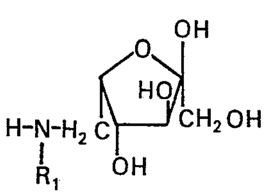

. HX (II)

worin

$R_1$ die obengenannte Bedeutung hat und HX eine starke anorganische oder organische Säure ist, mit Dimethylaminoboran in Wasser, Wasser-Lösungsmittelgemischen oder in organischen Lösungsmitteln reduziert.

2. Verfahren nach Anspruch 1, dadurch gekennzeichnet, dass das organische Lösungsmittel ein niederer Alkohol oder Ether ist.

3. Verfahren nach Anspruch 2, dadurch gekennzeichnet, dass der Alkohol Methanol, Ethanol oder Isopropanol ist.

4. Verfahren nach den Ansprüchen 1 bis 3, dadurch gekennzeichnet, dass die Reduktion bei Temperaturen von 0 bis 100°C durchgeführt wird.

5. Verfahren nach den Ansprüchen 1 bis 4, dadurch gekennzeichnet, dass das Molverhältnis zwischen den Verbindungen der allgemeinen Formel II und dem Dimethylaminoboran mindestens 1:0,25 beträgt.

6. Verfahren nach den Ansprüchen 1 bis 5, dadurch gekennzeichnet, dass bei der Reduktion basische Hilfsstoffe zur Bindung der Säure HX zugegen sind.

## Claims

1. Process for the preparation of 1,5-didesoxy-1,5-imino-D-glucitol and N-derivatives thereof of the general formula I

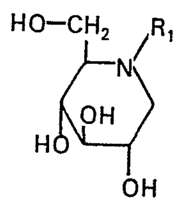

(I)

wherein

$R_1$ represents hydrogen, an alkyl radical with 1 to 30 C atoms, which can optionally be substituted by hydroxyl, alkoxy with 1 to 4 C atoms, acyloxy, the acyl radical being derived from aliphatic carboxylic acids with 1 to 7 C atoms, aromatic carboxylic acids, particularly phenylcarboxylic acid, which can be substituted in the phenyl radical by hydroxyl, halogen, $C_1$- to $C_4$-alkyl, $C_1$- to $C_4$-alkoxy, nitro and amino, and heterocyclic carboxylic acids derived from 5-membered or 6-membered heterocyclic structures, which contain 1 to 3 hetero-atoms from the series comprising N, O and S, and which can be substituted in the heterocyclic ring by $C_1$- to $C_4$-alkyl, chlorine, bromine and amino; amino, monoalkylamino and dialkylamino with preferably 1 to 4 carbon atoms in each alkyl radical, monoacylamino, acyl having the abovementioned meaning, mercapto, $C_1$- to $C_4$-alkylthio, halogen, $C_1$- to $C_4$-alkylcarbonyl, carboxyl, nitro, cyano, formyl, sulpho, heterocyclyl of the abovementioned definition, $C_3$- to $C_7$-cycloalkyl, and phenyl which is optionally substituted by halogen, $C_1$- to $C_4$-alkyl, $C_1$- to $C_4$-alkoxy, nitro, cyano or carboxyl, or an alkenyl radical, alkinyl radical or alkadienyl radical with 2 to 18 C atoms, characterised in that compounds of the general formula II

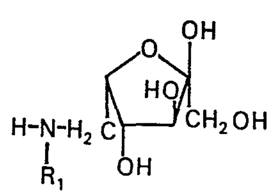

x HX (II)

wherein

$R_1$ has the above-mentioned meaning and HX is a strong inorganic or organic acid,

are reduced with dimethylaminoborane in water, water-solvent mixtures or in organic solvents.

2. Process according to Claim 1, characterised in that the organic solvent is a lower alcohol or ether.

3. Process according to Claim 2, characterised in that the alcohol is methanol, ethanol or isopropanol.

4. Process according to Claims 1 to 3, characterised in that the reduction is carried out at temperatures of 0 to 100°C.

5. Process according to Claims 1 to 4, characterised in that the molar ratio between the compounds of the general formula II and the dimethylaminoborane is at least 1:0.25.

6. Process according to Claims 1 to 5, characterised in that basic auxiliary materials for binding the acid HX are present in the reduction.

**Revendications**

1. Procédé de préparation de 1,5-didésoxy-1,5-imino-D-glucitol et de ses N-dérivés de formule générale I:

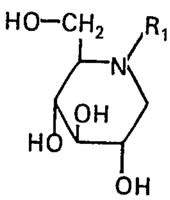

(I)

dans laquelle

$R_1$ représente de l'hydrogène, un radical alcoyle ayant 1 à 30 atomes de carbone, qui peut éventuellement être substitué par un hydroxy, alcoxy ayant 1 à 4 atomes de carbone, acyloxy, le radical acyle provenant d'acides carboxyliques aliphatiques ayant 1 à 7 atomes de carbone, d'acides carboxyliques aromatiques, en particulier d'acide phénylcarboxylique, qui peuvent être substitués dans le radical phényle par un hydroxy, halogène, alcoyle en $C_1$-$C_4$, alcoxy en $C_1$-$C_4$, nitro et amino, d'acides carboxyliques hétérocycliques qui dérivent d'hétérocycles à 5 ou 6 chaînons, qui contiennent 1 à 3 hétéroatomes de la série N, O et S et qui peuvent être substitués dans le noyau hétérocyclique par un alcoyle en $C_1$-$C_4$, du chlore, brome et amino; un amino, mono- et dialcoylamino ayant de préférence 1 à 4 atomes de carbone dans chaque radical alcoyle, monoacylamino, acyle ayant la signification citée plus haut, mercapto, alcoylthio en $C_1$-$C_4$, halogène, alcoylcarbonyle en $C_1$-$C_4$, carboxy, nitro, cyano, formyle, sulfo, hétérocyclyle de définition citée ci-dessus, cycloalcoyle en $C_3$-$C_7$, et phényle éventuellement substitué par de l'halogène, alcoyle en $C_1$-$C_4$, alcoxy en $C_1$-$C_4$, nitro, cyano ou carboxy, ou un radical alcényle, alcinyle ou alcadiényle ayant 2 à 18 atomes de carbone,

caractérisé en ce qu'on réduit des composés de formule générale II

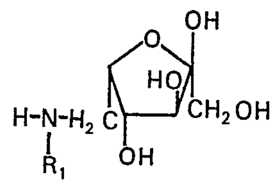 x HX (II)

dans laquelle $R_1$ a la signification citée plus haut et HX est un acide fort minéral ou organique, avec du diméthylaminoborane dans de l'eau, dans des mélanges eau-solvant ou dans des solvants organiques.

2. Procédé selon la revendication 1, caractérisé en ce que le solvant organique est un alcool inférieur ou un éther.

3. Procédé selon la revendication 2, caractérisé en ce que l'alcool est du méthanol, de l'éthanol ou de l'isopropanol.

4. Procédé selon les revendications 1 à 3, caractérisé en ce que la réduction est exécutée à des températures de 0 à 100°C.

5. Procédé selon les revendications 1 à 4, caractérisé en ce que le rapport molaire entre les composés de formule générale II et le diméthylaminoborane s'élève au moins à 1:0,25.

6. Procédé selon les revendications 1 à 5, caractérisé en ce qu'on ajoute dans la réduction des auxiliaires basiques pour la fixation de l'acide HX.